# EUROPEAN PATENT APPLICATION

(11) **EP 4 389 001 A1**
(43) Date of publication of application: **26.06.2024**
(21) Application number: 22216214.1
(22) Date of filing: 22.12.2022
(51) Int. Cl.: A61B 5/1455, A61B 5/1495, A61B 5/00

(54) **ALIGNING SIMULTANEOUSLY ACQUIRED IMAGES**

(71) Applicant: Neko Health AB, 115 45 Stockholm (SE)
(72) Inventor: WINDÅ, Mattias, 185 39 Vaxholm (SE); JOHANSSON, Henrik, 115 45 Stockholm (SE); RÖÖS, Magnus, 755 33 Uppsala (SE); TINGSTAM, Olle, 183 56 Täby (SE); ARVIDSSON, Magnus, 192 70 Sollentuna (SE)
(74) Representative: Kransell & Wennborg KB

(57) **Abstract**

A method comprises, with a predetermined pattern, irradiating an area of a sample (1) with electromagnetic radiation (4). The method also comprises, by each of a plurality of cameras simultaneously, during the irradiating of the sample with the predetermined pattern, acquiring an image of the sample area in a wavelength range specific to that camera. The method also comprises irradiating the sample area with a calibration pattern detectable by each of the cameras. The method also comprises each of the cameras detecting the calibration pattern from its respective view of the sample area. The method also comprises, based on the detecting of the calibration pattern by each of the cameras, determining rules for transforming respective images acquired by the cameras from the respective views to a primary view. The method also comprises aligning the acquired images of the sample area by using the determined rules.

## Description

### TECHNICAL FIELD

The present disclosure relates to a method of aligning images of a sample.

### BACKGROUND

EP 2 501 288 discloses an apparatus for determining subsurface optical properties of a sample of turbid media over an area of the sample. The apparatus comprises a source configured to expose the area of the sample to a plurality of structured illuminations, a detection system configured to collect optical signals emitted from the surface of the illuminated area, and a signal processor operably coupled to the detection system and configured to reconstruct optical data from the collected optical signals corresponding to the plurality of structured illuminations, wherein the collected optical signals from which optical data is reconstructed includes only one optical signal per each of the plurality of structured illuminations.

However, to determine the subsurface optical properties, optical signals at different wavelength ranges need to be collected, by using optical filters in front of a CCD camera, e.g. on a filter wheel.

### SUMMARY

It has now been realized that to acquire sequential images of a sample at different wavelengths takes long time, increasing the risk of the sample moving between images during the acquiring, especially if the sample is live tissue. It is herein proposed to instead acquire images at different respective wavelengths simultaneously by a plurality of cameras, e.g. arranged side by side in a detector. Then, however, each camera has a slightly different view of the sample, resulting in a problem of aligning the acquired images. Since each sample may have a distinct three-dimensional shape, this aligning may need to be specific for each sample.

According to an aspect of the present invention, there is provided a method of aligning simultaneously acquired images of a sample irradiated with a predetermined pattern. The method comprises, with said predetermined pattern, irradiating an area of the sample with electromagnetic radiation, typically including wavelengths absorbable by molecules present within the sample, below a surface of the sample. The method also comprises, by each of a plurality of cameras simultaneously, during the irradiating of the sample with the predetermined pattern, acquiring an image of the sample area in a wavelength range specific to that camera. The method also comprises irradiating the sample area with a calibration pattern detectable by each of the cameras. The method also comprises, during the irradiating with the calibration pattern, each of the cameras detecting the calibration pattern from its respective view of the sample area. The method also comprises, based on the detecting of the calibration pattern by each of the cameras, determining rules for transforming respective images acquired by the cameras from the respective views to a primary view. The method also comprises aligning the acquired images of the sample area by using the determined rules.

According to another aspect of the present invention, there is provided an imaging device comprising a controller comprising processing circuitry, and storage storing instructions executable by said processing circuitry whereby said imaging device is operative to perform an embodiment of the method of the present disclosure. The imaging device also comprises an irradiator for irradiating the sample with electromagnetic radiation, and a detector comprising the cameras.

According to another aspect of the present invention, there is provided a computer program product comprising computer-executable components for causing an imaging device, e.g. an embodiment of the imaging device of the present disclosure, to perform an embodiment of the method of the present disclosure when the computer-executable components are run on processing circuitry comprised in the imaging device.

By means of the calibration pattern, rules for transforming the images acquired of the sample from the respective sample views of the cameras can be determined and then used for aligning the acquired images of the sample when irradiated with the predetermined pattern. The rules may thus be specific for the sample, accounting for its three-dimensional shape. By the aligning, a point on the surface of the sample, preferably any point, may be identified in each of the acquired images, allowing aligning of the images at that point.

It is to be noted that any feature of any of the aspects may be applied to any other aspect, wherever appropriate. Likewise, any advantage of any of the aspects may apply to any of the other aspects. Other objectives, features and advantages of the enclosed embodiments will be apparent from the following detailed disclosure, from the attached dependent claims as well as from the drawings.

Generally, all terms used in the claims are to be interpreted according to their ordinary meaning in the technical field, unless explicitly defined otherwise herein. All references to "a/an/the element, apparatus, component, means, step, etc." are to be interpreted openly as referring to at least one instance of the element, apparatus, component, means, step, etc., unless explicitly stated otherwise. The steps of any method disclosed herein do not have to be performed in the exact order disclosed, unless explicitly stated. The use of "first", "second" etc. for different features/components of the present disclosure are only intended to distinguish the features/components from other similar features/components and not to impart any order or hierarchy to the features/components.

### BRIEF DESCRIPTION OF THE DRAWINGS

Embodiments will be described, by way of example, with reference to the accompanying drawings, in which:
Fig 1 is a schematic side view of an imaging device, in accordance with some embodiments of the present invention.
Fig 2 is a schematic bottom view of a detector of an imaging device, in accordance with some embodiments of the present invention.
Fig 3 is a schematic block diagram of a controller of an imaging device, in accordance with some embodiments of the present invention.
Fig 4 is an example image of a predetermined pattern irradiated onto a sample and acquired by a camera in a detector, in accordance with some embodiments of the present invention.
Fig 5 is an example image of a calibration pattern irradiated onto a sample and acquired by a camera in a detector, in accordance with some embodiments of the present invention.
Fig 6 is a schematic flow chart of a method, in accordance with some embodiments of the present invention.

### DETAILED DESCRIPTION

Embodiments will now be described more fully hereinafter with reference to the accompanying drawings, in which certain embodiments are shown. However, other embodiments in many different forms are possible within the scope of the present disclosure. Rather, the following embodiments are provided by way of example so that this disclosure will be thorough and complete, and will fully convey the scope of the disclosure to those skilled in the art. Like numbers refer to like elements throughout the description.

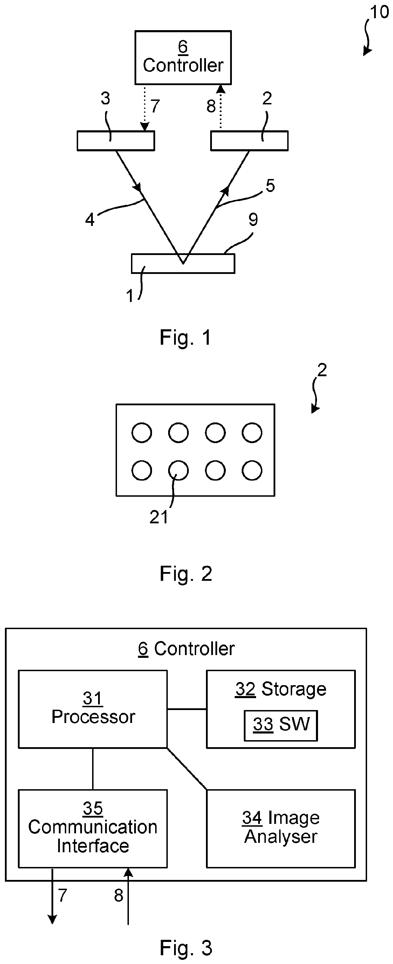

Figure 1 illustrates an imaging device 10 comprising an irradiator 3 and a detector 2. The irradiator may typically be a light source, e.g. comprising a plurality of Light-Emitting Diodes (LED), for instance emitting light 4 in the visible spectrum (VIS) with wavelengths from 380 nm to 750 nm and/or in the near-infrared spectrum (NIR) with wavelengths from 750 nm to 1400 nm. However, other wavelengths of electromagnetic radiation 4 may additionally or alternatively be emitted by the irradiator 3. For instance, at least five or at least eight LEDs may be comprised in the irradiator 3 for emitting the light 4, each with a respective different range of emitted wavelengths. The irradiator 3 preferably emits electromagnetic radiation, preferably VIS and NIR light, covering all the respective wavelength ranges of the cameras 21 in the detector 2 (see figure 2). A projector may be comprised in the irradiator, for projecting the predetermined and calibration patterns onto the sample. To study the inside of the sample 1, and not only the surface 9 thereof, it may be desirable to only detect, by the detector 2, radiation 5 which has been reflected by diffuse reflection, some distance below the surface 9 of the sample, and not detect any radiation reflected by specular reflection at the surface 9. In some embodiments, the irradiator 3 is configured to irradiate the sample 1 with polarized light, e.g. linearly polarized. Since specular reflected radiation keeps its polarization, it may be removed, by a polarization filter or the like, before reaching the sensors, e.g. CCD sensors, of the cameras 21 of the detector 2.

The sample 1 may be a tissue sample, e.g. a human or animal tissue sample, preferably a human tissue sample. In some embodiments, the tissue sample 1 is a sample of live tissue, e.g. part of an arm or leg of a human subject, preferably of a forearm of a human subject.

The imaging device 10 also comprises a controller 6 for controlling the apparatus 10 and for analysing the images acquired by the cameras 21 of the detector 2. Thus, the controller 6 may control the irradiating with predetermined and calibration patterns by the irradiator 3 by sending control signals 7 to the irradiator 3, and the controller may receive image signals 8, comprising the digital images acquired the detector during the irradiating. The controller 6 is further discussed with reference to figure 3.

Figure 2 illustrates a detector 2, e.g. as discussed with reference to figure 1. The detector comprises a plurality of cameras 21, e.g. an array of cameras 21. Since the cameras 21 are arranged beside each other, each has a slightly different view of the sample 1, why the aligning of the present invention is desirable in order to determine where in respective images of the different cameras a point of the sample 1, e.g. a point on the surface 9, is found. Any number of cameras 21 may be comprised in the detector 2, e.g. within the range of 4-10 cameras 21, such as eight cameras as in the figure.

Each camera 21 is configured to detect radiation within a predefined wavelength range specific for that camera, typically distinct or not overlapping with respective wavelength ranges of the other cameras in the detector 2. The radiation detected by each camera is, or at least includes, electromagnetic radiation from diffuse reflection in the sample 1 as a result of the irradiating by the irradiator 3. The specific wavelength ranges may include at least one VIS wavelength range and/or at least one NIR wavelength range, preferably at least one VIS wavelength range and at least one NIR wavelength range. In some embodiments, the specific wavelength ranges include a green VIS wavelength range, a red VIS wavelength range, and at least one NIR wavelength range. To achieve these respective specific wavelength ranges of the cameras 21, each camera may be provided with a bandpass filter corresponding to its specific wavelength range. In some embodiments, the respective bandpass filters of the cameras 21 include bandpass filter(s) having a nominal centre-wavelength value of: 532 nm, 560 nm, 580 nm, 589 nm, 650 nm, 725 nm, 850 nm and/or 975 nm.

As mentioned above, the irradiating by the irradiator 3 may be by polarized light, whereby radiation reflected by specular reflection in the surface 9 of the sample 1 may be removed by a polarization filter, e.g. a polarization filter per camera 21. Thus, in some embodiments, each camera 21 is provided with a respective polarization filter.

In an example, all LEDs of the irradiator 3 radiate simultaneously when irradiating the sample 1 with the predetermined and calibration patterns. With the help of mirrors that reflect or transmit depending on wavelength, a common optical beam path may be formed. The beam may be polarized, typically by linear polarization, and shines on a DMD (digital micromirror device - a projector) which is programmed to reflect the predetermined or calibration pattern. A projector lens focuses the pattern on the sample 1. Each camera 21 has a bandpass filter to acquire images in a specific wavelength range, as well as a polarizing filter to remove specular reflection.

In some embodiments, at least one infrared (IR) camera may be included in the detector 2, e.g. as one of the plurality of camera 21 simultaneously acquiring an image of the sample area. IR may be defined as wavelengths of from 1,4 micrometres (µm) to 15 µm. However, it may not be possible to project the calibration pattern using IR. In that case, the IR camera 21 may include both an IR sensor and a visible light (VIS), or possibly NIR, sensor to form a camera arrangement comprising both an IR sensor and a VIS or NIR sensor. Alternatively, the camera arrangement comprises both an IR camera and a VIS or NIR camera arranged close together, e.g. integrated with each other, such that the IR camera and the VIS/NIR camera has substantially, or at least close to, the same view of the sample 1. In the camera arrangement, the IR sensor may be used for obtaining an IR image of the sample, e.g. during the irradiating with the predetermined pattern, while the VIS/NIR sensor may be used for detecting the calibration pattern for obtaining the rules for aligning the IR image with the respective images acquired by the (other) cameras 21 (e.g. VIS and/or NIR cameras). For instance, the smallest distance between respective lens edges of the lenses for the IR sensor and the VIS/NIR sensor may be at most 1 cm. Thus, in some embodiments of the present invention, at least one of the cameras 21 comprises an IR sensor for acquiring an image in an infrared wavelength range and provides a camera arrangement which also comprises a VIS (or NIR) sensor for detecting the calibration pattern. The IR image may be a simultaneously acquired image 40, acquired during the irradiating of the sample 1 with the predetermined pattern. However, additionally or alternatively, IR image(s) of the sample may be acquired by means of the IR sensor before and/or after the irradiating of the sample with the predetermined pattern. To acquire an IR image before or after, especially before, the irradiating with the predetermined pattern may be suitable to ensure that the IR image captures the naturally radiated heat from the sample and is not affected by the irradiating with the predetermined pattern. In some embodiments, an objective of acquiring an IR image is to look at the naturally radiated heat from the sample 1. If the sample is not irradiated with IR, the acquired IR image may not be impacted by the lower-wavelength (VIS and/or NIR) irradiated predetermined patterns.

Figure 3 illustrates an embodiment of the controller 6. The controller 6 comprises processing circuitry 31 e.g. a central processing unit (CPU). The processing circuitry 31 may comprise one or a plurality of processing units in the form of microprocessor(s). However, other suitable devices with computing capabilities could be comprised in the processing circuitry 31, e.g. an application specific integrated circuit (ASIC), a field programmable gate array (FPGA) or a complex programmable logic device (CPLD). The processing circuitry 31 is configured to run one or several computer program(s) or software (SW) 33 stored in a storage 32 of one or several storage unit(s) e.g. a memory. The storage unit is regarded as a computer readable means 32, forming a computer program product together with the SW 33 stored thereon as computer-executable components and may e.g. be in the form of a Random Access Memory (RAM), a Flash memory or other solid state memory, or a hard disk, or be a combination thereof. The processing circuitry 31 may also be configured to store data in the storage 32, as needed. The controller 6 may also comprise an image analyser 34, e.g. for determining the rules for transforming the images from different cameras to common primary view, and to apply those rules to align the acquired images with each other. The controller 6 may also comprise a communication interface 35, e.g. for communicating with other parts of the imaging device 10. For example, the controller may send control signals 7 via the communication interface 35 and/or receive image signals 8 via the communication interface 35.

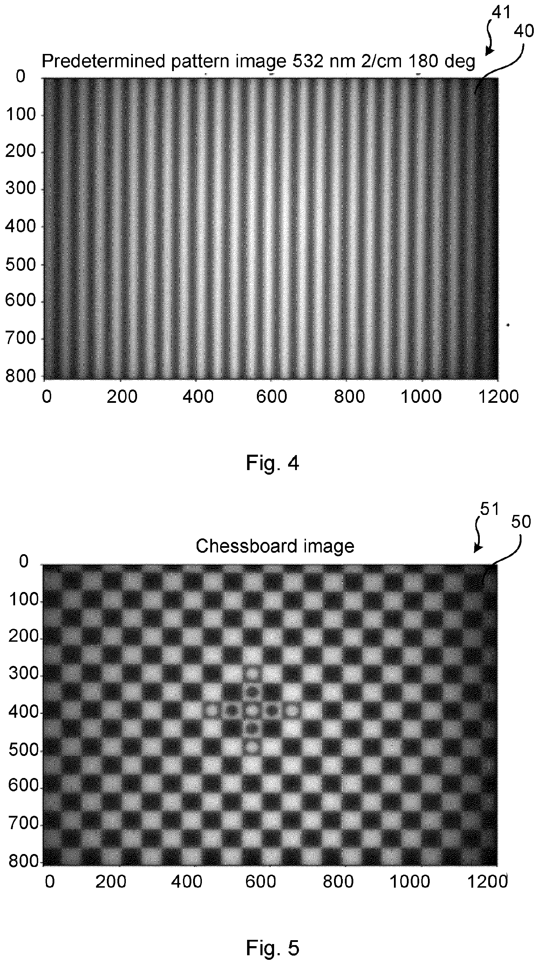

Figure 4 shows an example of an acquired image 40 of a predetermined pattern 41 projected onto the sample 1. The unit on the axes are pixel indices (same for figure 5), whereby in this example row 400 and column 600 is approximately the middle of the image. In this example, a sinusoidal pattern is used. To illuminate the whole sample area, a plurality of predetermined patterns 41 may be needed, e.g. with different phase offsets of the sinusoidal pattern, such as 60°, 180° and 300° offsets, where the pattern 41 having 180° offset is shown in the image 40 of the figure. In addition to its offset, the sinusoidal pattern 41 may also be defined by its spatial frequency, where the pattern 41 of the image 40 in the figure has a frequency corresponding to two wavelengths (or spatial periods) per centimetre (cm) at a standard distance of the sample from the irradiator/projector 3, when irradiating a planar surface 9 of the sample 1 at a right angle. In the example of figure 4, the image 40 is acquired by a camera 21 having a bandpass filter with a nominal centre-wavelength value of 532 nanometres (nm).

Figure 5 shows an example of an acquired image 50 of a calibration pattern 50, here in the form of a chessboard pattern. The calibration pattern is projected onto the sample with radiation including wavelengths such that the pattern is detectable by all of the cameras 21 in the detector 2. The calibration pattern 51 is configured such that it is possible to align images of the pattern from each of the different cameras, from their respective views of the sample area. For instance, the calibration pattern may include a feature which is readily detectable and may serve as an origin for determining coordinates within the respective images 50. In the example of figure 5, a central cross with circles serves as such a feature within the chessboard pattern 51. Starting from the origin, coordinates may be assigned to other features in the calibration pattern 51, e.g. corners in the chessboard pattern of figure 5. Optionally, a distinct ID may be assigned to each feature/corner. Each respective image 50 of the calibration pattern 51 detected by the cameras 21 is analysed to identify the features therein, e.g. with coordinates or IDs. Then, rules, specific for the sample 1, can be determined for transforming respective images 50 or 40 acquired by each camera 21 from its view of the sample to a common primary view. Then, the determined rules can be used for aligning the images 40 of the predetermined pattern 41, e.g. comprising Piecewise Affine Transform.

For example, coordinates in the calibration pattern 51, in the form of a chessboard pattern as in figure 5, correspond to the corner locations in the chessboard pattern, so there is a certain spacing between coordinates, which may e.g. be >10 pixels in the images 50. Therefore, there are typically not corresponding coordinates for each pixel in each camera view, why there may be a need to approximate a transform between coordinates. The piecewise affine transform may perform adequately for this. In theory, however, coordinates for every pixel in the image 50 may be determined, allowing each pixel in the acquired images 40 to be aligned with each other.

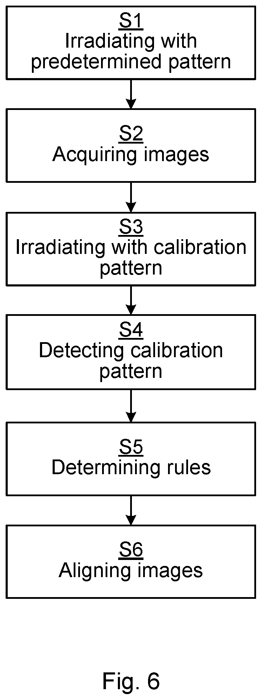

Figure 6 illustrates some embodiments of the method of the present disclosure. The method is for aligning simultaneously acquired images 40 of the sample 1 while it is irradiated with a predetermined pattern 41 projected onto the sample. An area of the sample 1 is irradiated S1 with electromagnetic radiation 4. In some embodiments, the radiation 4 includes wavelengths absorbable by molecules present within the sample, below a surface 9 of the sample. The sample may be a tissue sample, in which case the molecules may be chromophores present in tissue, such as water, melanin, oxygenated haemoglobin and/or unoxygenated haemoglobin. Typically, the predetermined pattern 41 is static during the acquiring of images thereof. During the irradiating S1 of the sample 1 with the predetermined pattern 41, an image 40 of the sample area is acquired S2 by each of a plurality of cameras 21 simultaneously in a wavelength range specific to that camera. Before or after the irradiating S1 with the predetermined pattern, the sample area is irradiated S3 with a calibration pattern 51 detectable by each of the cameras 21. The calibration patten may be projected onto a surface 9 of the sample. The calibration pattern is then detected S4, e.g. by acquiring an image thereof, by each of the cameras 21 from its respective view of the sample area (resulting from its viewing angle of the sample) during the irradiating S3 with the calibration pattern 51. Thus, the same pattern is detected S4 in parallel by the cameras 21. Based on the detecting S4 of the calibration pattern 51 by each of the cameras 21, rules are determined S5 for transforming the respective images 40, acquired S2 by the cameras 21, from the respective views to a primary view. Then, the determined S5 rules are applied to align S6 the acquired S2 images 40 of the sample area.

The present disclosure has mainly been described above with reference to a few embodiments. However, as is readily appreciated by a person skilled in the art, other embodiments than the ones disclosed above are equally possible within the scope of the present disclosure, as defined by the appended claims.

## Claims

1. A method of aligning simultaneously acquired images (40) of a sample (1) irradiated with a predetermined pattern (41), the method comprising:
with said predetermined pattern (41), irradiating (S1) an area of the sample (1) with electromagnetic radiation (4) including wavelengths absorbable by molecules present within the sample, below a surface (9) of the sample;
by each of a plurality of cameras (21) simultaneously, during the irradiating (S1) of the sample (1) with the predetermined pattern (41), acquiring (S2) an image (40) of the sample area in a wavelength range specific to that camera;
irradiating (S3) the sample area with a calibration pattern (51) detectable by each of the cameras (21);
during the irradiating (S3) with the calibration pattern (51), each of the cameras (21) detecting (S4) the calibration pattern from its respective view of the sample area;
based on the detecting (S4) of the calibration pattern (51) by each of the cameras (21), determining (S5) rules for transforming respective images (40) acquired (S2) by the cameras from the respective views to a primary view; and
aligning (S6) the acquired (S2) images (40) of the sample area by using the determined (S5) rules.

2. The method of claim 1, wherein the sample (1) is a tissue sample, e.g. a human or animal tissue sample, preferably a human tissue sample.

3. The method of claim 2, wherein the tissue sample (1) is a live tissue sample.

4. The method of any preceding claim, wherein the aligning (S6) comprises Piecewise Affine Transform.

5. The method of any preceding claim, wherein the plurality of cameras (21) are within the range of 4-10 cameras, preferably eight cameras.

6. The method of any preceding claim, wherein the respective specific wavelength ranges of the cameras (21) include at least one wavelength range in the visual spectrum, VIS, and at least one wavelength range in the near-infrared spectrum, NIR.

7. The method of any preceding claim, wherein each of the cameras (21) is provided with a bandpass filter corresponding to its specific wavelength range.

8. The method of claim 7, wherein the respective bandpass filters of the cameras (21) include bandpass filter(s) having a nominal centre-wavelength value of: 532 nm, 560 nm, 580 nm, 589 nm, 650 nm, 725 nm, 850 nm and/or 975 nm.

9. The method of any preceding claim, wherein the plurality of cameras (21) are comprised in a detector (2) which also comprises at least one camera arrangement comprising both an IR sensor for acquiring an IR image of the sample area and a VIS or NIR, preferably VIS, sensor for detecting the calibration pattern (51).

10. The method of any preceding claim, wherein the irradiating (S1) with the predetermined pattern (41) and the irradiating (S3) with the calibration pattern (51) is by a plurality of LEDs emitting electromagnetic radiation (4) in respective different wavelength ranges.

11. The method of any preceding claim, wherein the irradiating (S1) with the predetermined pattern (41) and the irradiating (S3) with the calibration pattern (51) is by polarized light (4).

12. The method of claim 11, wherein at least one, e.g. some or all, of the cameras (21) is provided with a polarization filter.

13. An imaging device (10) comprising:
a controller (6) comprising processing circuitry (31), and storage (32) storing instructions (33) executable by said processing circuitry whereby said imaging device is operative to perform the method of any preceding claim;
an irradiator (3) for irradiating the sample (1) with electromagnetic radiation (4); and
a detector (2) comprising the cameras (21).

14. A computer program product (32) comprising computer-executable components (33) for causing an imaging device (10) to perform the method of any one of claims 1-12 when the computer-executable components are run on processing circuitry (31) comprised in the imaging device.
